# EUROPÄISCHE PATENTANMELDUNG

(11) **EP 2 570 111 A1**
(43) Veröffentlichungstag der Anmeldung: **20.03.2013**
(21) Anmeldenummer: 12183184.6
(22) Anmeldetag: 05.09.2012
(51) Int. Cl.: A61K 8/42, A61K 8/46, A61Q 5/02, A61Q 19/10, C07C 233/18, C11D 1/65, C11D 1/94, C11D 11/00

(54) **Kontinuierliches Herstellungsverfahren on Tensidmischungen**

(30) Priorität: 15.09.2011 DE 102011082760
(71) Anmelder: Henkel AG&Co. KGAA, 40589 Düsseldorf (DE)
(72) Erfinder: Schröder, Thomas, 22395 Hamburg (DE); Hentrich, Dirk, 22457 Hamburg (DE); Wechsler, Michael, 91734 Mitteleschenbach (DE); Bauer, Reinhold, 91174 Spalt (DE); Meyer, Richard, 91726 Gerolfingen (DE)

(57) **Zusammenfassung**

Die Erfindung betrifft ein Verfahren zur Herstellung einer Tensid(vor)mischung A, das die folgenden Verfahrensschritte aufweist:
I) Aufschmelzen einer Verbindung der nachfolgenden Formel (i) 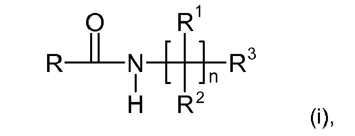 in der
- der Rest R für einen geradkettigen oder verzweigten, gesättigten oder ein- oder mehrfach ungesättigten Alkyl- oder Alkenlyrest mit 8 bis 24 Kohlenstoffatomen steht,
- die Reste R¹, R² und R³ unabhängig voneinander für ein Wasserstoffatom, eine Hydroxylgruppe oder eine C₁-C₄-Alkylgruppe stehen, und
- der Index n für eine der Zahlen 2, 3 oder 4 steht,

wobei mindestens einer der Reste R¹, R² und R³ eine Hydroxylgruppe bedeutet und wobei nicht zwei Hydroxylgruppen gleichzeitig an ein Kohlenstoffatom gebunden sind,
II) Hinzufügen eines 2- bis 8-fachen Überschusses an Wasser einer Temperatur > 65°C und Vermischen des Wassers mit der in Verfahrensschritt I) hergestellten Reaktionsmischung,
III) Hinzufügen mindestens eines - von (i) verschiedenen - Tensids (ii), ausgewählt aus
- Alkylsulfat- und/oder Alkylpolyglycolethersulfatsalzen und/oder
- amphoteren/zwitterionischen Tensiden der nachfolgenden Formel

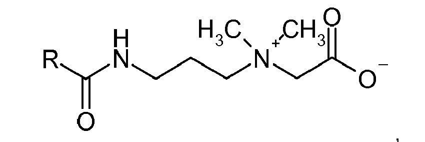

in der der Rest R für einen geradkettigen oder verzweigten, gesättigten oder ein- oder mehrfach ungesättigten Alkyl- oder Alkenlyrest mit 8 bis 24 Kohlenstoffatomen steht, und
Vermischen des (der) Tensids(e) (ii) mit der Reaktionsmischung, die in Verfahrensschritt II) hergestellt wurde,
IV) Hinzufügen von Wasser (ad 100) einer Temperatur von 20°C +/- 5°C, mit der Maßgabe, dass das Gewichtsverhältnis der Verbindung(en) nach Formel (i) zu dem (den) Tensid(en) (ii) 1 : 6 bis 2 : 1 beträgt.

## Beschreibung

Die Erfindung liegt auf dem Gebiet der Kosmetik und betrifft ein Verfahren zur Herstellung einer speziellen Tensid(vor)mischung sowie die Verwendung der Tensid(vor)mischung zur Herstellung kosmetischer Reinigungsmittel.

Gebrauchsfertige Tensidmischungen und/oder Tensidkonzentrate sind bekannt und im Handel erhältlich. Sie werden hergestellt, um Transportkosten und Lagerkapazitäten zu reduzieren, und um als sogenannte Basismischung für die Herstellung einer Vielzahl kosmetischer Mittel zu dienen.

Fettsäurealkanolamide können in kosmetischen Mitteln als Schaumverstärker, Verdickungsmittel und/oder Stabilisatoren eingesetzt werden. Aufgrund ihrer positiven Eigenschaften - insbesondere in kosmetischen Reinigungsmitteln - ist man daher bestrebt Tensidmischungen herzustellen, die neben den klassischen Reinigungstensiden auch Fettsäurealkanolamide enthalten.

Problematisch bei der Verarbeitung der Fettsäurealkanolamide ist, dass sie vor der Durchmischung mit anderen Komponenten (wie auch Wasser) üblicherweise in separaten Apparaturen aufgeschmolzen werden müssen.

Dieser Prozess erfordert einen erhöhten Zeit-, Kosten- und Energieaufwand und weist zudem den Nachteil auf, dass in finalen Formulierungen (in denen separat aufgeschmolzene Fettsäurealkanolamide verarbeitet wurden) Probleme bei der Schaumbildung und der Viskosität auftreten können.

Darüber hinaus können insbesondere bei der üblichen Dosierung der flüssigen Fettsäurealkanolamide durch Pumpen oftmals unerwünschte Lufteinschlüsse in den wässrigen Tensidmischungen auftreten.

Im Handel erhältliche Tensidmischungen weisen den Nachteil auf, dass sie Fettsäurealkanolamide meist nur in geringen Mengen enthalten. Es werden auch Mischungen mit höheren Mengen an Fettsäurealkanolamiden angeboten, doch ist deren Viskosität meist so hoch, dass sie bei der Herstellung eines Reinigungsmittels nicht direkt eingesetzt werden können. Eine vorherige Verdünnung der Mischungen ist zunächst erforderlich, für die große Zwischentanks eingesetzt werden müssen, was die Verarbeitung der Mischungen unökonomisch macht.

Ziel der vorliegenden Erfindung war daher die zuvor genannten Nachteile zu überwinden und ein verbessertes Herstellungsverfahren für Fettsäurealkanolamide-enthaltende Tensidmischungen zu entwickeln.

Insbesondere sollten die resultierenden Tensidmischungen größere Mengen an Fettsäurealkanolamiden enthalten, ohne eine zu hohe Viskosität aufzuweisen.

Ein erster Gegenstand der vorliegenden Anmeldung ist ein Verfahren zur Herstellung einer Tensid(vor)mischung A, bei dem die folgenden Verfahrensschritte durchlaufen werden:
I) Aufschmelzen einer Verbindung der nachfolgenden Formel (i) 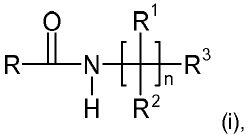 in der
   - der Rest R für einen geradkettigen oder verzweigten, gesättigten oder ein- oder mehrfach ungesättigten Alkyl- oder Alkenlyrest mit 8 bis 24 Kohlenstoffatomen,
   - die Reste R¹, R² und R³ unabhängig voneinander für ein Wasserstoffatom, eine Hydroxylgruppe oder eine C₁-C₄-Alkylgruppe, und
   - der Index n für eine der Zahlen 2, 3 oder 4 steht,
   wobei mindestens einer der Reste R¹ R² und R³ eine Hydroxylgruppe bedeutet und wobei nicht zwei Hydroxylgruppen gleichzeitig an ein Kohlenstoffatom gebunden sind,
II) Hinzufügen eines 2- bis 8-fachen Überschusses an Wasser einer Temperatur > 65°C und Vermischen des Wassers mit der in Verfahrensschritt I) hergestellten Reaktionsmischung,
III) Hinzufügen mindestens eines - von (i) verschiedenen - Tensids (ii), ausgewählt aus
   - Alkylsulfat- und/oder Alkylpolyglycolethersulfatsalzen und/oder
   - amphoteren/zwitterionischen Tensiden der nachfolgenden Formel 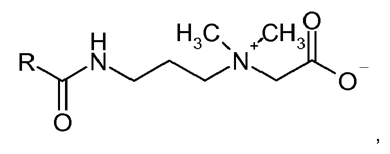 in der der Rest R für einen geradkettigen oder verzweigten, gesättigten oder ein - oder mehrfach ungesättigten Alkyl- oder Alkenlyrest mit 8 bis 24 Kohlenstoffatomen steht, und
   Vermischen des (der) Tensids(e) (ii) mit der Reaktionsmischung, die in Verfahrensschritt II) hergestellt wurde,
IV) Hinzufügen von Wasser (ad 100) einer Temperatur von 20°C +/- 5°C,
   mit der Maßgabe, dass das Gewichtsverhältnis der Verbindung(en) nach Formel (i) zu dem (den) Tensid(en) (ii) 1 : 6 bis 2 : 1 beträgt.

In einer ersten bevorzugten Ausführungsform des erfindungsgemäßen Verfahrens folgt den Verfahrensschritten I) bis IV) ein weiterer Verfahrensschritt V), in dem der Tensid(vor)mischung A eine oder mehrere Puffersubstanzen und/oder eine oder mehrere Substanzen zur pH-Wert-Einstellung und/oder eine oder mehrere Substanzen zur Konservierung zugegeben werden.

Geeignete Puffersubstanzen können beispielsweise aus Essigsäure/Acetatpuffern, Citronensäure/Citratpuffern, Kohlensäure/Bicarbonatpuffern und/oder Milchsäure/Lactatpuffern ausgewählt sein.

Als Substanzen zur pH-Wert-Einstellung können prinzipiell alle physiologisch verträglichen Säuren und/oder (je nach pH-Wert) Basen eingesetzt werden.

Bevorzugt sind NaOH, Alkanolamine, Citronensäure, Milchsäure, Weinsäure, Äpfelsäure und/oder Sorbinsäure.

Geeignete Konservierungsmittel können ausgewählt sein aus Benzoesäure und Sorbinsäure sowie den physiologisch verträglichen Salzen und Estern dieser Säuren, Phenoxyethanol, Benzylakohol, Parabenen, Chlorbutanol, Dehydracetsäure, Bronidox^{®}, Bronopol^{®}, Triclosan^{®}, Chlorhexidin, Methylisothiazoline und/oder DMDM Hydantoine^{®}.

Bevorzugt sind Benzoesäure oder Benzoesäuresalze wie beispielsweise Natriumbenzoat, Methylisothiazoline, Phenoxyethanol, Benzylalkohol und/oder DMDM Hydantoin^{®}.

Die zuvor genannten Puffersubstanzen, Substanzen zur pH-Wert-Einstellung und/oder Konservierungsmittel können der durch das erfindungsgemäße Verfahren erhältlichen Tensid(vor)mischung A jeweils in Mengen von 0,005 bis 5 Gew.-%, bevorzugt von 0,01 bis 3 Gew.-% und insbesondere von 0,05 bis 2 Gew.-% zugegeben werden, wobei sich die Mengenangaben auf das Gesamtgewicht der Tensid(vor)mischung A beziehen.

Bevorzugte Verbindungen der Formel (i), die in Verfahrensschritt I) aufgeschmolzen werden können, weisen bevorzugt einen Schmelzpunkt im Bereich von 40 bis 95°C, mehr bevorzugt von 45 bis 85°C und insbesondere von 50 bis 80°C auf.

Besonders bevorzugte Verbindungen der Formel (i) weisen
- als Rest R geradkettige oder verzweigte, gesättigte oder ein- oder mehrfach ungesättigte Alkyl- oder Alkenlyreste mit 8 bis 18 Kohlenstoffatomen und
- als Index n die Zahlen 2 oder 3 auf, wobei
mindestens einer der Reste R¹, R² und R³ eine Hydroxylgruppe bedeutet und wobei nicht zwei Hydroxylgruppen gleichzeitig an ein Kohlenstoffatom gebunden sind.

Ganz besonders bevorzugte Verbindungen der Formel (i) sind Monoethanol- und/oder Monoisopropanolfettsäureamide, wie beispielsweise die unter den INCI-Bezeichnungen Cocamide MEA und Cocamide MIPA bekannten und von verschiedenen Anbietern im Handel erhältlichen Verbindungen.

Verbindungen der Formel (i) können in dem erfindungsgemäßen Verfahren bevorzugt in Mengen von 2 bis 20 Gew.-%, mehr bevorzugt von 2,5 bis 17,5 Gew.-%, weiter bevorzugt von 3 bis 15 Gew.-% und insbesondere von 4 bis 10 Gew.-% eingesetzt werden, wobei sich die Mengenangaben auf das Gesamtgewicht der Tensid(vor)mischung A beziehen.

Geeignete Tensid(e) (ii) können ausgewählt sein aus Alkylsulfat- und/oder Alkylpolyglykolethersulfatsalzen und/oder amphoteren/zwitterionischen Tensiden der Formel 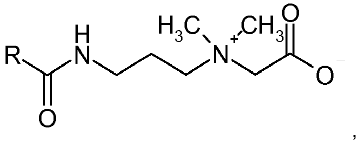 in der R für einen geradkettigen oder verzweigten, gesättigten oder ein- oder mehrfach ungesättigten Alkyl- oder Alkenlyrest mit 8 bis 24 Kohlenstoffatomen steht.

Bevorzugte Alkylsulfat- und/oder Alkylpolyglykolethersulfatsalze entsprechen bevorzugt der Formel R-O(CH₂-CH₂O)ₓ-OSO₃⁻ X⁺, in der R bevorzugt für eine lineare oder verzweigte, gesättigte oder ungesättigte Alkylgruppe mit 8 bis 30 C-Atomen, x für 0 oder eine Zahl von 1 bis 12 und X für ein Alkali-, ein Erdalkali-, ein Ammonium- oder ein Alkanolaminion steht.

Besonders bevorzugt sind geradkettige oder verzweigte Alkylethersulfate, die einen Alkylrest mit 8 bis 18 und insbesondere mit 10 bis 16 C-Atomen, sowie 1 bis 6 und insbesondere 2 bis 4 Ethylenoxideinheiten aufweisen.

Insbesondere bevorzugt sind die Natrium-, Magnesium und/oder Triethanolaminsalze linearer oder verzweigter Lauryl-, Tridecyl- und/oder Myristylsulfate, die einen mittleren Ethoxylierungsgrad von 2 bis 4 aufweisen.

Das oder die Alkylsulfat- und/oder Alkylpolyglykolethersulfatsalz(e) können in dem erfindungsgemäßen Verfahren bevorzugt in Mengen von 1 bis 20 Gew.-%, mehr bevorzugt von 2 bis 18 Gew.-%, besonders bevorzugt von 4 bis 17 Gew.-% und insbesondere von 5 bis 15 Gew.-% eingesetzt werden, wobei sich die Mengenangaben auf das Gesamtgewicht der Tensid(vor)mischung A beziehen. Bevorzugte amphotere und/oder zwitterionische Tenside der zuvor genannten Formel enthalten als Rest R überwiegend einen geradkettigen oder verzweigten, gesättigten oder ein- oder mehrfach ungesättigten Alkylrest mit 8 bis 20, mehr bevorzugt von 8 bis 16 und insbesondere mit 8 bis 12 C-Atomen.

Besonders bevorzugte amphotere/zwitterionische Tenside sind C₈-C₁₂-Alkylamido(C₁-C₄)alkylbetaine, wie beispielsweise die unter den INCI-Bezeichnungen Lauramidopropylbetain und/oder Cocamidopropylbetain bekannten Tenside.

Insbesondere bevorzugt ist ein unter der INCI-Bezeichnung Cocamidopropylbetain bekanntes und im Handel von mehreren Anbietern erhältliches amphoteres Tensid.

Das oder die amphotere(n)/zwitterionische(n) Tensid(e) können in dem erfindungsgemäßen Verfahren bevorzugt in Mengen von 1 bis 20 Gew.-%, mehr bevorzugt von 2 bis 18 Gew.-%, besonders bevorzugt von 4 bis 17 Gew.-% und insbesondere von 5 bis 15 Gew.-% eingesetzt werden, wobei sich die Mengenangaben auf das Gesamtgewicht der Tensid(vor)mischung A beziehen.

In einer zweiten bevorzugten Ausführungsform werden die Komponente(n) (i) und (ii) in dem erfindungsgemäßen Verfahren in einem Gewichtsverhältnis von 1 : 5 bis 1,5 : 1, bevorzugt von 1 : 4 bis 1,25 : 1 und insbesondere von 1 : 3 bis 1 : 1 eingesetzt.

Die aus dem erfindungsgemäßen Verfahren resultierenden Tensid(vor)mischungen A müssen ausreichend viskos sein, damit die Komponente (i) darin stabilisiert bleibt. Gleichzeitig sollte die Viskosität der Tensidmischungen A nicht zu hoch sein, damit eine problemlose Weiterverarbeitung gewährleistet ist.

In einer dritten bevorzugten Ausführungsform weist die aus dem erfindungsgemäßen Verfahren resultierende Tensid(vor)mischung A daher bevorzugt eine Viskosität im Bereich von 10 bis 10,000 mPas, mehr bevorzugt von 20 bis 7,500 mPas, besonders bevorzugt von 30 bis 5,000 mPas und insbesondere von 50 bis 4,000 mPas auf (jeweils gemessen mit einem Haake Rotationsviskosimeter VT550; 20°C; Meßeinrichtung MV; Spindel MV II; 8 UPM).

Die durch das erfindungsgemäße Verfahren erhältliche Tensid(vor)mischung A sollte dazu geeignet sein als Basis für eine Vielzahl kosmetischer Reinigungsmittel zu dienen.

Um dieses Ziel zu erreichen darf der pH-Wert der Tensid(vor)mischung nicht allzu niedrig sein, da sich anderenfalls negative Wechselwirkungen mit anderen Komponenten bei der Weiterverarbeitung der Tensid(vor)mischung A ergeben können.

In einer vierten bevorzugten Ausführungsform weist die Tensid(vor)mischung A einen pH-Wert ≥ 4, mehr bevorzugt ≥ 4,25 und insbesondere ≥ 4,5 auf.

Ein wesentlicher Vorteil des erfindungsgemäßen Verfahrens besteht darin, dass variable Mengen der Stoffe (i) und (ii) in einem kontinuierlichen Heiß-Kalt-Dosierungsprozess verarbeitet werden können.

Durch das erfindungsgemäße Verfahren können demnach bedarfsorientiert sowohl Fettsäurealkanolamid-enthaltende Tensidvormischungen zur späteren Weiterverarbeitung hergestellt werden (es handelt sich dann um ein Premix), als auch finale Reinigungszusammensetzungen.

Zur Herstellung einer finalen Reinigungszusammensetzungen können der Vormischung A in einem oder mehreren Schritten weitere kosmetische Inhaltsstoffe zugegeben werden.

In einer fünften bevorzugten Ausführungsform wird der Tensidvormischung A in einem zusätzlichen Verfahrensschritt VI) weiterhin mindestens eine Komponente aus einer der folgenden Gruppen der:
➢ anionischen, nichtionischen und/oder amphoteren/zwitterionischen Tenside,
➢ kationischen Polymere,
➢ Proteinhydrolysate,
➢ Öl-, Fett- und/oder Wachskomponenten,
➢ Perlglanzmittel und/oder Trübungsmittel und/oder
➢ Vitamine/Vitaminderivate und/oder Vitaminvorstufen
zugegeben.

Innerhalb dieser Ausführungsform ist es besonders bevorzugt, wenn der Tensidvormischung A in einem weiteren Verfahrensschritt VI) mindestens ein weiteres Tensid, ausgewählt aus der Gruppe der anionischen, nichtionischen und/oder amphoteren/zwitterionischen Tenside zugegeben wird. Bei den zusätzlichen anionischen und/oder amphoteren/zwitterionischen Tensiden kann es sich auch um diejenigen Tenside handeln, die bereits in der Vormischung A als Tensidkomponente (ii) eingesetzt wurde(n).

Zu den geeigneten anionischen Tensiden, die in mindestens einem zusätzlichen Verfahrensschritt VI) eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓCH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓOSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 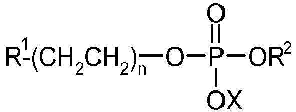 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ -Kohlenwasserstoffrest, steht.

Bevorzugte zusätzliche anionische Tenside sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Das oder die zusätzlichen anionischen Tensid(e) können in dem mindestens einen zusätzlichen Verfahrensschritt VI) bevorzugt in Mengen von 3 bis 20 Gew.-%, besonders bevorzugt von 5 bis 15 Gew.-% und insbesondere von 7 bis 12 Gew.-% eingesetzt werden, wobei sich die Mengenangaben auf das Gesamtgewicht des finalen Reinigungsmittels beziehen.

Geeignete amphotere/zwitterionische Tenside, die in mindestens einem zusätzlichen Verfahrensschritt VI) eingesetzt werden können, sind bevorzugt ausgewählt aus Verbindungen der folgenden Formeln 1 bis 5, in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,

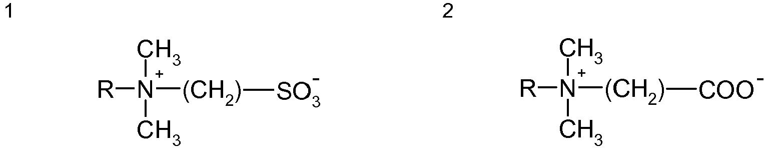

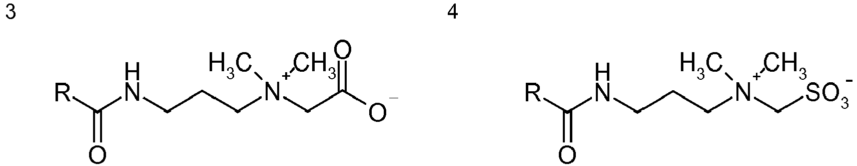

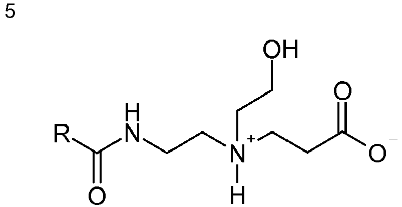

Besonders geeignete amphotere/zwitterionische Tenside sind Alkylamidoalkylbetaine und/oder Alkylampho(di)acetate der zuvor genannten Formeln 1 bis 5.

Zu den insbesondere geeigneten amphoteren/zwitterionischen Tensiden zählen die unter der INCI-Bezeichnung Cocamidopropylbetain und/oder Disodium Cocoampho(di)acetate bekannten Tenside.

Das oder die zusätzlichen amphoteren/zwitterionischen Tensid(e) können in dem mindestens einen zusätzlichen Verfahrensschritt VI) bevorzugt in Mengen von 0,5 bis 15 Gew.-%, besonders bevorzugt von 0,75 bis 10 Gew.-% und insbesondere von 1 bis 7,5 Gew.-% eingesetzt werden, wobei sich die Mengenangaben auf das Gesamtgewicht des finalen Reinigungsmittels beziehen.

Zu den geeigneten nichtionischen Tensiden/Emulgatoren, die in mindestens einem zusätzlichen Verfahrensschritt VI) eingesetzt werden können, zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettamine und/oder
- Alkylpolyglucoside.

Besonders geeignete nichtionische Tenside sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, und Fettsäurealkanolamide, insbesondere C₈-C₂₄-Fettsäure(di)ethanolamide.

Weiterhin bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

Das oder die zusätzlichen nichtionischen Tensid(e)/Emulgatoren können in dem mindestens einen zusätzlichen Verfahrensschritt VI) bevorzugt in Mengen von 0,1 bis 10 Gew.-%, besonders bevorzugt von 0,25 bis 7,5 Gew.-% und insbesondere von 0,5 bis 5 Gew.-% eingesetzt werden, wobei sich die Mengenangaben auf das Gesamtgewicht des finalen Reinigungsmittels beziehen.

Kosmetische Reinigungsmittel enthalten üblicherweise neben Tensiden auch mindestens eine pflegende Komponente, damit die gereinigte Haut und/oder die gereinigten Haare während der Reinigung nicht zu stark strapaziert werden.

Innerhalb der fünften bevorzugten Ausführungsform ist es daher insbesondere bevorzugt, wenn der Tensidvormischung A in mindestens einem weiteren Verfahrensschritt neben mindestens einem weiteren Tensid auch mindestens eine Pflegekomponente, ausgewählt aus einer der folgenden Gruppen der:
➢ kationischen Polymere,
➢ Proteinhydrolysate,
➢ Öl-, Fett- und/oder Wachskomponenten,
➢ Perlglanzmittel und/oder Trübungsmittel und/oder
➢ Vitamine/Vitaminderivate und/oder Vitaminvorstufen
zugegeben wird.

Geeignete kationische Polymere, die in dem mindestens einen zusätzlichen Verfahrensschritt eingesetzt werden können, sind beispielsweise:
- quaternisierte Cellulose-Derivate, wie sie unter den Bezeichnungen Celquat^{®} und Polymer JR^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Be zeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte kationische Polymere sind quaternisierte Cellulosepolymere, kationische Guarderivate und/oder kationische Polymere auf Acrylsäure(derivat)basis, die besonders bevorzugt ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67.

Das oder die kationische(n) Polymer(e) kann (können) in dem mindestens einen zusätzlichen Verfahrensschritt bevorzugt in einer Menge 0,01 bis 5 Gew.-%, besonders bevorzugt von 0,05 bis 3 Gew.-% und insbesondere von 0,1 bis 2 Gew.-% eingesetzt werden, wobei sich die Mengenangaben auf das Gesamtgewicht des finalen Reinigungsmittels beziehen.

Geeignete Proteinhydrolysate, die in dem mindestens einen zusätzlichen Verfahrensschritt eingesetzt werden können, sind vorzugsweise pflanzlichen, tierischen oder marinen Ursprungs. Sie können in dem mindestens einen zusätzlichen Verfahrensschritt bevorzugt in einer Menge von 0,01 bis 10 Gew.-%, mehr bevorzugt von 0,25 bis 7,5 Gew.-% und insbesondere von 0,05 bis 5 Gew.-% eingesetzt werden, wobei sich die Mengenangaben auf das Gesamtgewicht des finalen Reinigungsmittels beziehen.

Geeignete tierische Proteinhydrolysate sind beispielsweise Elastin-, Kollagen-, Keratin-, Seiden - und/oder Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Geeignete Proteinhydrolysate pflanzlichen Ursprungs sind beispielsweise Soja-, Mandel-, Reis-, Erbsen-, Kartoffel-, Raps- und/oder Weizenproteinhydrolysate.

Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Zu den geeigneten Proteinhydrolysaten marinen Ursprunges zählen beispielsweise Kollagenhydrolysate von Fischen oder Algen sowie Proteinhydrolysate von Muscheln bzw. Perlenhydrolysate. Beispiele für geeignete Perlenhydrolysate sind die Handelsprodukte Pearl Protein Extract BG^{®} oder Crodarom^{®} Pearl.

Einsetzbar sind auch kationisierte Proteinhydrolysate, wobei das zugrunde liegende Proteinhydrolysat aus den zuvor beschriebenen tierischen, pflanzlichen und/oder marinen Quellen stammen kann. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternärer Ammoniumsalze wie beispielsweise N,N-Dimethyl-N-(n-Alkyl)-N-(2-hydroxy-3-chloro-n-propyl)-ammoniumhalogeniden durchgeführt.

Weiterhin können die kationischen Proteinhydrolysate auch noch weiter derivatisiert sein. Als typische Beispiele für geeignete kationische Proteinhydrolysate und/oder -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Geeignete Öl- und/oder Fettkomponenten, die in dem mindestens einen zusätzlichen Verfahrensschritt eingesetzt werden können, sind bevorzugt ausgewählt aus mineralischen, natürlichen und synthetischen Ölkomponenten und/oder Fettstoffen.

Sie können in dem mindestens einen zusätzlichen Verfahrensschritt bevorzugt in einer Menge von 0,001 bis 10 Gew.-%, mehr bevorzugt von 0,005 bis 7,5 Gew.-% und insbesondere von 0,01 bis 5 Gew.-% eingesetzt werden, wobei sich die Mengenangaben auf das Gesamtgewicht des finalen Reinigungsmittels beziehen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S). Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-undecylether sowie Di-tert.-butylether, Di-isopentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol^{®} OE erhältlich ist.

Als synthetische Öle kommen Silikonverbindungen in Betracht.

Geeignete Silikone (die sich von dem Silikon a) unterscheiden) können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®}871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau. Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM),
   Isononansäure-C16-18-alkylester (Cetiol^{®} SN), 2-Ethylhexylpalmitat (Cegesoft^{®} 24), Stearinsäure-2-ethylhexylester (Cetiol^{®}868), Cetyloleat, Glycerintricaprylat, Kokosfettalkohol- caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Unter geeigneten Perlglanzmitteln und Trübungsmitteln, die in dem mindestens einen zusätzlichen Verfahrensschritt eingesetzt werden können, sind beispielsweise
- Mono- und/oder Diester des Ethylenglycols, 1,2-Propandiols und/oder Glycerins mit C₈-C₂₄-Fettsäuren,
- Ester aus Polyethylenglycolen mit C₈-C₂₄-Fettsäuren und/oder
- Styrol/Acrylat-Copolymere zu verstehen.

Sie können in dem mindestens einen zusätzlichen Verfahrensschritt bevorzugt in einer Menge von 0,001 bis 5 Gew.-%, mehr bevorzugt von 0,005 bis 4 Gew.-%, besonders bevorzugt von 0,01 bis 3 Gew.-% und insbesondere von 0,05 bis 2 Gew.-% eingesetzt werden, wobei sich die Mengenangaben auf das Gesamtgewicht des finalen Reinigungsmittels beziehen.

Besonders geeignet sind die unter den INCI-Bezeichnungen bekannten Trübungs- und/oder Perlglanzmittel:
Glycol Distearate, wie beispielsweise das Handelsprodukt Cutina^{®} AGS der Firma Cognis, Glycol Monostearate, wie beispielsweise das Handelsprodukt Cutina^{®} EGMS der Firma Cognis, PEG-3 Distearate, wie beispielsweise das Handelsprodukt Genapol^{®} TS der Firma Clariant, PEG-2 Distearate, wie beispielsweise das Handelsprodukt Kessco^{®} DEGMS der Firma Akzo Nobel, Propylen Glycol Stearate, wie beispielsweise das Handelsprodukt Tegin^{®} P der Firma Goldschmidt und/oder Styrol/Acrylates-Copolymere wie beispielsweise die Handelsprodukte Joncryl^{®} 67 der Firma Johnson Polymers, Suprawal^{®} WS der Firma BASF und/oder Acusol^{®} OP 301 der Firma Rohm & Haas. Insbesondere geeignet sind die unter den INCI-Bezeichnungen bekannten Trübungs- und/oder Perlglanzmittel: Glycol Distearate, Glycol Monostearate, PEG-3 Distearate und/oder Styrol/Acrylates-Copolymer.

Unter geeigneten Vitaminen, die in dem mindestens einen zusätzlichen Verfahrensschritt eingesetzt werden können, sind bevorzugt die folgenden Vitamine, Provitamine und Vitaminvorstufen sowie deren Derivate zu verstehen:
● Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
● Vitamin B: zur Vitamin B-Gruppe oder zu dem Vitamin B-Komplex gehören u. a.
   o Vitamin B₁ (Thiamin)
   o Vitamin B₂ (Riboflavin)
   o Vitamin B₃. Unter dieser Bezeichnung werden häufig die Verbindungen Nicotinsäure und Nicotinsäureamid (Niacinamid) geführt.
   o Vitamin 8₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   o Vitamin B₆ (Pyridoxin sowie Pyridoxamin und Pyridoxal).
● Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
● Vitamin E (Tocopherole, insbesondere α-Tocopherol).
● Vitamin F: unter dem Begriff "Vitamin F" werden üblicherweise essentielle Fettsäuren, insbesondere Linolsäure, Linolensäure und Arachidonsäure, verstanden.
● Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol.

Die Vitamine, Vitaminderivate und/oder Vitaminvorstufen können in dem mindestens einen zusätzlichen Verfahrensschritt bevorzugt in einer Menge von 0,001 bis 2 Gew.-%, besonders bevorzugt von 0,005 bis 1 Gew.-% und insbesondere von 0,01 bis 0,5 Gew.-% eingesetzt werden, wobei sich die Mengenangaben auf das Gesamtgewicht des finalen Reinigungsmittels beziehen.

Das erfindungsgemäße Verfahren weist den Vorteil auf, dass variable Mengen der Stoffe (i) und (ii) in einem kontinuierlichen Heiß-Kalt-Dosierungsprozess verarbeitet werden können, wodurch die Effizienz des Herstellungsprozesses gesteigert werden kann.

Durch das erfindungsgemäße Verfahren können bedarfsorientiert sowohl Fettsäurealkanolamid-enthaltende Tensidvormischungen zur späteren Weiterverarbeitung hergestellt werden (es handelt sich dann um ein Premix), als auch finale Reinigungszusammensetzungen. Dadurch können Lager- und Transportkosten eingespart werden.

Die aus dem erfindungsgemäßen Verfahren erhältlichen Tensid(vor)mischungen weisen geeignete Viskositäten auf, um als Basis für eine Vielzahl kosmetischer Reinigungsmittel zu dienen. So eignet sich das erfindungsgemäße Verfahren auch für Batch-Ansätze >10t und kontinuierliche Dosierungsprozesse.

Gegenüber einem Verfahren zur Herstellung von Fettsäurealkanolamiden-enthaltenden Tensidmischungen, in denen die Fettsäurealkanolamide in einem separaten Schmelzprozess aufgeschmolzen und anschließend über Pumpen der wässrigen Tensidmischung zugeführt werden, ist das erfindungsgemäße Verfahren ebenfalls vorteilhaft, denn unerwünschte Schaumbildung und/oder Lufteinschlüsse konnten in dem erfindungsgemäßen Verfahren durch die Durchmischung der erforderlichen Komponenten in einem geschlossenen System weitestgehend verhindert werden.

Ein zweiter Gegenstand der Erfindung ist die Verwendung der aus dem erfindungsgemäßen Verfahren resultierenden Tensid(vor)mischung A zur Herstellung eines kosmetischen Reinigungsmittels.

Ein dritter Gegenstand der Erfindung ist ein kosmetisches Reinigungsmittel, das in einem geeigneten Träger
- mindestens eine nach dem erfindungsgemäßen Verfahren hergestellte Tensid(vor)mischung A sowie
- mindestens eine Komponente aus einer der folgenden Gruppen der:
   ➢ anionischen, nichtionischen und/oder amphoteren/zwitterionischen Tenside,
   ➢ kationischen Polymere,
   ➢ Proteinhydrolysate,
   ➢ Öl-, Fett- und/oder Wachskomponenten,
   ➢ Perlglanzmittel und/oder Trübungsmittel und/oder
   ➢ Vitamine/Vitaminderivate und/oder Vitaminvorstufen enthält.

Unter einem geeigneten Träger wird bevorzugt ein wässriger oder wässrig-alkoholischer Träger verstanden.

Bevorzugt enthält der Träger mindestens 50 Gew.-%, mehr bevorzugt mindestens 60 Gew.-% und besonders bevorzugt mindestens 70 Gew.-% Wasser.

Weiterhin kann der Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Hinsichtlich der Erläuterung der weiteren Inhaltsstoffe des kosmetischen Reinigungsmittels wird auf die entsprechenden Absätze zum erfindungsgemäßen Verfahren an früherer Stelle der Beschreibung verwiesen.

Neben den bereits früher genannten obligatorischen und fakultativen Inhaltsstoffen können die kosmetischen Reinigungsmittel einen oder mehrere weitere Wirk-, Hilfs- und Zusatzstoffe aus der folgenden Gruppe der:
- UV-Filter,
- Strukturanten wie Maleinsäure und Milchsäure,
- Farbstoffe zum Anfärben des Mittels,
- Hautberuhigenden Wirkstoffe wie Allantoin und/oder Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Parfums,
- Pflanzenextrakte,
- Moisturizing-Komponenten,
- Antischuppenmittel wie Zinkpyrithion, Piroctone Olamine, Salicylsäure und/oder Ketokonazole enthalten.

### Beispiele:

I. Im Folgenden wird die Herstellung verschiedener Varianten von Tensidvormischungen mithilfe des erfindungemäßen Verfahrens beschrieben. Die Mengenangaben beziehen sich dabei (sofern nicht anders angegeben) auf Gew.-%.

| | Varianten | | | | | | |
|---|---|---|---|---|---|---|---|
| **Rohstoffe** | **1** | **2** | **3** | **4** | **5** | **6** | **7** |
| Wasser (70°C +/- 5°C) | 30,0 | 30,0 | 30,0 | 30,0 | 30,0 | 30,0 | 30,0 |
| Cocamide MEA | 5,0 | 7,5 | 7,5 | 10,0 | | 7,5 | |
| Cocamide MIPA | | | | | 7,5 | | 7,5 |
| Sodium Laureth Sulfate (25% AS) | 25,0 | 25,0 | 30,0 | 40,0 | 25,0 | | |
| Cocamidopropyl Betaine (40% AS) | | | | | | 30,0 | 30,0 |
| Wasser (20°C +/- 5°C) | ad 100 | ad 100 | ad 100 | ad 100 | ad 100 | ad 100 | ad 100 |
| Wasser (20°C +/- 5°C) | 2,0 | 2,0 | 2,0 | | | | |
| DMDM Hydantoin (10% AS) | | | | | 0,1 | 0,1 | |
| Methylisothiazoline | | | | 0,1 | | | 0,1 |
| Natrium Benzoate | 0,5 | 0,5 | 0,5 | | | | |
| Citronensäure | 0,1 | 0,1 | 0,1 | | | | |
| pH-Wert | 4,5-5,5 | 4,5-5,5 | 4,5-5,5 | 4,5-5,5 | 6,0-7,0 | 6,0-7,0 | 6,0-7,0 |
| Viskosität (bei 25°C)* | 200 | 3900 | 940 | 6100 | 150 | 850 | 720 |

| | | | | | | | |
|---|---|---|---|---|---|---|---|
| * Die Viskosität wurde jeweils gemessen mit einem Haake Rotationsviskosimeter VT550; 25°C; Meßeinrichtung MV; Spindel MV II; 8 UPM | | | | | | | |

Das genaue Herstellungsverfahren wird anhand der Variante 1 beschrieben. Die Verfahrensvarianten 2 bis 7 wurden analog durchgeführt.
1. Cocamide MEA wurde in einem Schmelzkessel mit geeignetem Rührwerk bei einer Temperatur aufgeschmolzen, die etwa 20% über dem Schmelzbereich des Cocamide MEA liegt (etwa 72 bis 77°C). Die Durchmischung der Schmelze erfolgt im oberen Drittel des Kessels; die Entnahme erfolgt am unteren Ende des Kessels.
2. Die nachfolgende Dosierung der flüssigen Komponenten erfolgt mittels geeigneter Dosierpumpen in der angegebenen Reihenfolge entlang einer Mischstrecke:
   a) Zugabe von heißem Wasser (70°C +/- 5°C) zur Schmelze,
   b) Zugabe des Tensids (SLES, 25%ig),
   c) Zugabe von kaltem Wasser (ad 100).
3. Die Zugabe zusätzlichen Wassers, eines Konservierungsmittels und eines Säuerungsmittels (Natriumbenzoat und Citronensäure) in einem zusätzlichen Verfahrensschritt ist fakultativ.)
4. Durchmischen bis zur Homogenität und vollständigen Abkühlung der Vormischung auf Raumtemperatur.
IIIm Folgenden wird die anschließende Weiterverarbeitung einer nach dem erfindungsgemäßen Verfahren hergestellten Tensidvormischung zu einer kosmetischen Reinigungszusammensetzung beschrieben. Die Mengenangaben beziehen sich dabei (sofern nicht anders angegeben) auf Gew.-%.

| Rohstoffgruppe | Rohstoff | Menge |
|---|---|---|
| 1 | Sodium Laureth Sulfate (25% AS) | 32,0 |
| 2a | Wasser | 3,0 |
| 2a | Sodium Laureth Sulfate (25% AS) | 2,0 |
| 2c | Farbstoff | 0,02 |
| 2c | Natrium Benzoate | 0,28 |
| 2c | Citronensäure | 0,28 |
| 2d | Parfumöl | 0,50 |
| 2d | PEG-40 Hydrogenated Castor Oil | 0,50 |
| 2c | DMDM Hydantoin (10% AS) | 0,05 |
| 3 | **5%ige Cocamide MEA-Phase** | 24,0 |
| 4 | Polyquaternium-10 | 0,25 |
| 4 | DMDM Hydantoin (10% AS) | 0,05 |
| 5 | Euperlan^{®1} PK 3000 | 4,0 |
| 6 | Cocamidopropyl Betaine (40% AS) | 5,5 |
| 7 | Citronensäure | 0,3 |
| 8 | NaCl | 0,4 |
| 9 | Wasser | ad 100 |

In dem Reinigungsmittel wurde das folgende Handelsprodukt eingesetzt:
1 INCI-Bezeichnung: Wasser, Glycol Distearate, Glycerin, Laureth-4, Cocamodopropyl Betaine, Formic Acid; BASF

### Herstellungsverfahren:

a) Die Rohstoffe der Gruppe 2a wurden bei etwa 20 bis 25°C vorgelegt und unter Rühren mit den Rohstoffen der Gruppe 2c vermischt. Die Rohstoffe der Gruppe 2d wurden separat vorgemischt und anschließend unter Rühren zur Mischung (2a, 2c) hinzugegeben.
b) Die Zugabe der Rohstoffmischung 2 zur Cocamide MEA-enthaltenden Vormischung 3 (vgl. Variante 1 unter Punkt I)sowie die Zugabe der restlichen Rohstoffe bzw. Rohstoffmischungen erfolgte bei Raumtemperatur in der angegebenen Reihenfolge auf einer Rohrmischstrecke.

## Patentansprüche

1. Verfahren zur Herstellung einer Tensid(vor)mischung A, **gekennzeichnet durch** die folgenden Verfahrensschritte:
I) Aufschmelzen einer Verbindung der nachfolgenden Formel (i) 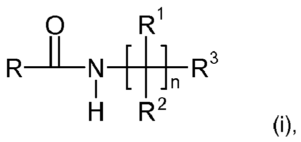 in der
- der Rest R für einen geradkettigen oder verzweigten, gesättigten oder ein- oder mehrfach ungesättigten Alkyl- oder Alkenlyrest mit 8 bis 24 Kohlenstoffatomen steht,
- die Reste R¹, R² und R³ unabhängig voneinander für ein Wasserstoffatom, eine Hydroxylgruppe oder eine C₁-C₄-Alkylgruppe stehen, und
- der Index n für eine der Zahlen 2, 3 oder 4 steht,
wobei mindestens einer der Reste R¹ R² und R³ eine Hydroxylgruppe bedeutet und wobei nicht zwei Hydroxylgruppen gleichzeitig an ein Kohlenstoffatom gebunden sind,
II) Hinzufügen eines 2- bis 8-fachen Überschusses an Wasser einer Temperatur > 65°C und Vermischen des Wassers mit der in Verfahrensschritt I) hergestellten Reaktionsmischung,
III) Hinzufügen mindestens eines - von (i) verschiedenen - Tensids (ii), ausgewählt aus
- Alkylsulfat- und/oder Alkylpolyglycolethersulfatsalzen und/oder
- amphoteren/zwitterionischen Tensiden der nachfolgenden Formel 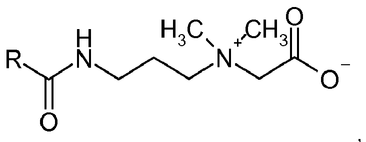 in der der Rest R für einen geradkettigen oder verzweigten, gesättigten oder ein - oder mehrfach ungesättigten Alkyl- oder Alkenlyrest mit 8 bis 24 Kohlenstoffatomen steht, und
Vermischen des (der) Tensids(e) (ii) mit der Reaktionsmischung, die in Verfahrensschritt II) hergestellt wurde,
IV) Hinzufügen von Wasser (ad 100) einer Temperatur von 20°C +/- 5°C, mit der Maßgabe, dass das Gewichtsverhältnis der Verbindung(en) nach Formel (i) zu dem (den) Tensid(en) (ii) 1 : 6 bis 2 : 1 beträgt.

2. Verfahren nach Anspruch 1, **dadurch gekennzeichnet, dass** der Tensid(vor)mischung A in einem zusätzlichen Verfahrensschritt V) eine oder mehrere Puffersubstanzen und/oder eine oder mehrere Substanzen zur pH-Wert-Einstellung und/oder eine oder mehrere Substanzen zur Konservierung zugegeben werden.

3. Verfahren nach einem der Ansprüche 1 oder 2, **dadurch gekennzeichnet, dass** in Verfahrensschritt I) mindestens eine Verbindung der Formel (i) aufgeschmolzen wird, die einen Schmelzpunkt im Bereich von 40 bis 95°C, bevorzugt von 45 bis 85°C und insbesondere von 50 bis 80°C aufweist.

4. Verfahren nach einem der Ansprüche 1 bis 3, **dadurch gekennzeichnet, dass** in Verfahrensschritt I) mindestens eine der unter den INCI-Bezeichnungen Cocoamide MEA und Cocamide MIPA bekannten Verbindungen (i) aufgeschmolzen wird.

5. Verfahren nach einem der Ansprüche 1 bis 4, **dadurch gekennzeichnet, dass** in Verfahrensschritt III) als Tensid(e) (ii) mindestens ein geradkettiges oder verzweigtes Alkylethersulfat, das einen Alkylrest mit 8 bis 18 und insbesondere mit 10 bis 16 C-Atomen sowie 1 bis 6 und insbesondere 2 bis 4 Ethylenoxideinheiten aufweist und/oder mindestens ein C₈-C₁₂-Alkylamido(C₁-C₄)alkylbetain hinzugefügt wird (werden).

6. Verfahren nach einem der Ansprüche 1 bis 5, **dadurch gekennzeichnet, dass** die Tensid(vor)mischung A eine Viskosität im Bereich von 10 bis 10,000 mPas, bevorzugt von 20 bis 7,500 mPas, besonders bevorzugt von 30 bis 5,000 mPas und insbesondere von 50 bis 4,000 mPas aufweist (jeweils gemessen mit einem Haake Rotationsviskosimeter VT550; 20°C; Meßeinrichtung MV; Spindel MV II; 8 UPM).

7. Verfahren nach einem der Ansprüche 1 bis 6, **dadurch gekennzeichnet, dass** die Tensid(vor)mischung A einen pH-Wert ≥ 4 aufweist.

8. Verfahren nach einem der Ansprüche 1 bis 7, **dadurch gekennzeichnet, dass** der Tensid(vor)mischung A in einem zusätzlichen Verfahrensschritt VI) weiterhin mindestens eine Komponente aus einer der folgenden Gruppen der:
➢ anionischen, nichtionischen und/oder amphoteren/zwitterionischen Tenside,
➢ kationischen Polymere,
➢ Proteinhydrolysate,
➢ Öl-, Fett- und/oder Wachskomponenten,
➢ Perlglanzmittel und/oder Trübungsmittel und/oder
➢ Vitamine/Vitaminderivate und/oder Vitaminvorstufen
zugegeben wird.

9. Verwendung einer nach einem der Ansprüche 1 bis 7 hergestellten Tensid(vor)mischung A zur Herstellung eines kosmetischen Reinigungsmittels.

10. Kosmetisches Reinigungsmittel, enthaltend in einem geeigneten Träger
- mindestens eine nach einem der Ansprüche 1 bis 7 hergestellten Tensid(vor)mischung A sowie
- mindestens eine Komponente aus einer der folgenden Gruppen der:
➢ anionischen, nichtionischen und/oder amphoteren/zwitterionischen Tenside,
➢ kationischen Polymere,
➢ Proteinhydrolysate,
➢ Öl-, Fett- und/oder Wachskomponenten,
➢ Perlglanzmittel und/oder Trübungsmittel und/oder
➢ Vitamine/Vitaminderivate und/oder Vitaminvorstufen.
